# EUROPÄISCHE PATENTSCHRIFT

(11) **EP 3 485 272 B1**
(45) Veröffentlichungstag und Bekanntmachung des Hinweises auf die Patenterteilung: **24.04.2024**
(21) Anmeldenummer: 17740368.0
(22) Anmeldetag: 12.07.2017
(51) Int. Cl.: G01N 33/02

(54) **VERFAHREN ZUR ÜBERPRÜFUNG DES MINDESTHALTBARKEITSDATUMS EINES VERPACKTEN PRODUKTES**
METHOD FOR CHECKING THE BEST BEFORE DATE OF A PACKAGED PRODUCT
PROCÉDÉ DE VÉRIFICATION DE LA DATE LIMITE D'UTILISATION OPTIMALE D'UN PRODUIT EMBALLÉ

(30) Priorität: 15.07.2016 DE 102016213010
(43) Veröffentlichungstag der Anmeldung: 22.05.2019
(73) Patentinhaber: GEA Food Solutions Germany GmbH, 35216 Biedenkopf-Wallau (DE)
(72) Erfinder: STEFFEN, Andreas, 35116 Hatzfeld-Holzhausen (DE); MÜLLER, Sven, 35216 Biedenkopf (DE); SASSMANNSHAUSEN, Volker, 57334 Bad Laasphe (DE)
(74) Vertreter: Kutzenberger Wolff & Partner
(86) Internationale Anmeldenummer: PCT/EP2017/067622
(87) Internationale Veröffentlichungsnummer: WO 2018/011307

(56) Entgegenhaltungen:
- WO-A1-2010/145892
- WO-A1-2015/013030
- CN-A- 101 349 685
- ANDREAS HEMPEL ET AL: "Use of Optical Oxygen Sensors in Non-Destructively Determining the Levels of Oxygen Present in Combined Vacuum and Modified Atmosphere Packaged Pre-Cooked Convenience-Style Foods and the Use of Ethanol Emitters to Extend Product Shelf-Life", FOODS, Bd. 2, Nr. 4, 18. November 2013 (2013-11-18), Seiten 507-520, XP055410694, DOI: 10.3390/foods2040507
- T. P. LABUZA ET AL: "Growth kinetics for shelf-life prediction: Theory and practice", JOURNAL FOR INDUSTRIAL MICROBIOLOGY, Bd. 12, Nr. 3-5, September 1993 (1993-09), Seiten 309-323, XP055410229, UK ISSN: 0169-4146, DOI: 10.1007/BF01584208
- ULRIKE HERBERT ET AL: "Definition of predictor variables for MAP poultry filets stored under different temperature conditions", POULTRY SCIENCE, Bd. 94, Nr. 3, 30. Januar 2015 (2015-01-30), Seiten 424-432, XP055427456, Oxford ISSN: 0032-5791, DOI: 10.3382/ps/peu002
- LIMBO S ET AL: "Evaluation and predictive modeling of shelf life of minced beef stored in high-oxygen modified atmosphere packaging at different temperatures", MEAT SCIENCE, ELSEVIER SCIENCE, GB, Bd. 84, Nr. 1, 1. Januar 2010 (2010-01-01) , Seiten 129-136, XP026684012, ISSN: 0309-1740, DOI: 10.1016/J.MEATSCI.2009.08.035 [gefunden am 2009-08-20]
- ABAD ET AL: "Flexible tag microlab development: Gas sensors integration in RFID flexible tags for food logistic", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER BV, NL, Bd. 127, Nr. 1, 5. Oktober 2007 (2007-10-05), Seiten 2-7, XP022286071, ISSN: 0925-4005, DOI: 10.1016/J.SNB.2007.07.007
- XUEZHEN HONG ET AL: "Discrimination and prediction of multiple beef freshness indexes based on electronic nose", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER BV, NL, Bd. 161, Nr. 1, 19. Oktober 2011 (2011-10-19), Seiten 381-389, XP028447742, ISSN: 0925-4005, DOI: 10.1016/J.SNB.2011.10.048 [gefunden am 2011-11-07]
- EL BARBRI ET AL: "Building of a metal oxide gas sensor-based electronic nose to assess the freshness of sardines under cold storage", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER BV, NL, Bd. 128, Nr. 1, 7. November 2007 (2007-11-07), Seiten 235-244, XP022335667, ISSN: 0925-4005, DOI: 10.1016/J.SNB.2007.06.007

## Beschreibung

Die vorliegende Erfindung betrifft ein zur Überprüfung des Mindesthaltbarkeitsdatums eines verpackten Lebensmittels oder sensiblen Gutes.

Das MHD/Verbrauchsdatum/Einfrierdatum eines Lebensmittels oder eines sensiblen Produktes, wie eines Medizin- oder Kosmetikproduktes wird in der Regel aufgrund von Erkenntnissen aus der Mikrobiologie und/oder aufgrund von langjähriger Erfahrung mit dem jeweiligen Produkt oder ähnlichen bzw. vergleichbaren Produkten ermittelt. Da der Produzent garantieren muss, dass seine Ware bis zu diesem Datum nicht nur haltbar ist, sondern dass auch Konsistenz, Geschmack und Geruch im Wesentlichen erhalten bleiben, werden vergleichsweise kurze MHDs/Verbrauchsdaten für eine ganze Charge, beispielsweise eine Tagescharge angegeben, wodurch viele Lebensmittel oder sensible Produkte unnötigerweise zu früh aus dem Verkehr gezogen werden. Dies ist nicht nur wirtschaftlich problematisch, sondern stellt auch eine Verschwendung von Ressourcen dar.

WO2015013030 A1 beschreibt ein Verfahren zur Überprüfung des Mindesthaltbarkeitsdatums eines Lebensmittels, wobei Bedingungen innerhalb der Verpackung, ermittelt mittels eines internen Sensors, und Transport-und Lagerbedingungen, ermittelt mittels eines externen Sensors, berücksichtigt werden.

Es war deshalb die Aufgabe der vorliegenden Erfindung ein Verfahren zur Überprüfung des Mindesthaltbarkeitsdatums eines verpackten Lebensmittels oder sensiblen Gutes zur Verfügung zu stellen, das die Nachteile des Standes der Technik nicht aufweist.

Gelöst wird die Aufgabe mit einem Verfahren nach Anspruch 1.

Bei den Lebensmitteln handelt es sich vorzugsweise um in mikrobiologischer Hinsicht verderbliche Lebensmitteln und/oder Backwaren und/oder proteinhaltige Lebensmittel wie Wurst, Schinken oder Käse aber auch um Fleisch, insbesondere Schweine-, Rind-, Geflügel-, Wild- und/oder Lammfleisch, das prozessiert, beispielsweise zerkleinert, mariniert, paniert und/oder einer Wärmebehandlung und/oder Kältebehandlung und/oder Strahlungsbehandlung, beispielsweise einer UV-Behandlung, unterzogen worden sein kann. Dieses Verpackungsgut wird in einer Verpackung, insbesondere einer Verpackung bestehend aus einer oder mehreren Kunststofffolien, verpackt. Oftmals wird die Luft in der Verpackung zumindest teilweise durch ein anderes, insbesondere Inertgas wie N₂ und/oder CO₂ ersetzt, um die Mindesthaltbarkeit und/oder das Verbrauchsdatum des Lebensmittels oder sonstigen sensiblen Verpackungsgut zu verlängern und/oder den Einfrierzeitpunkt (Einfrierdatum) zu verschieben.

Das Mindesthaltbarkeitsdatum (MHD) ist eine beispielsweise bei Lebensmitteln und anderen sensiblen Verpackungsgütern, z. B. medizintechnischen oder kosmetischen Produkten, vorzugsweise vorgeschriebene, aber im Ermessen des Herstellers liegende Angabe, die angibt, bis zu welchem Zeitpunkt (Datum) beispielsweise ein Lebensmittel bei sachgerechter Aufbewahrung (insbesondere unter Einhaltung der Produkttemperatur während der gesamten Transportkette) ohne wesentliche Geschmacks- und Qualitätseinbußen sowie ohne Gesundheitsrisiko zu konsumieren ist. Das Lebensmittel ist in aller Regel auch nach dem angegebenen Zeitpunkt (Datum) noch verzehrbar und darf dann vorzugsweise noch verkauft werden. Die Angabe des MHD erfolgt beispielsweise durch "mindestens haltbar bis:" gefolgt von beispielsweise einer Datumsangabe. Alternativ oder zusätzlich wird das Verbrauchsdatum angegeben, nach dessen Ablauf beispielsweise vom Verkehr von dem Lebensmittel abgeraten wird und/oder nach dennoch erfolgtem Verzehr ein Gesundheitsrisiko bestehen kann. Das Lebensmittel darf nach Ablauf des Verbrauchsdatums vorzugsweise nicht mehr verkauft werden. Die Angabe des Verbrauchsdatums erfolgt beispielsweise durch "zu verbrauchen bis:" gefolgt von beispielsweise einer Datumsangabe. Zusätzlich zum MHD und/oder Verbrauchsdatum kann das Einfrierdatum angegeben werden, beispielsweise durch "eingefroren am:" gefolgt von beispielsweise einer Datumsangabe. MHD/Verbrauchsdatum/Einfrierdatum gelten üblicherweise nur für noch ungeöffnete Verpackungen und beziehen sich häufig auf eine konkrete durchgehende Produkttemperatur. Die vorliegende Erfindung bezieht sich gleichermaßen auch auf alternative Begriffe zu MHD/Verbrauchsdatum/Einfrierdatum, die im selben Kontext verwendet werden, also eine Haltbarkeitsangabe ähnlich wie MHD/Verbrauchsdatum oder ein Produktionsdatum ähnlich wie Einfrierdatum im Sinne der Erfindung darstellen.

Erfindungsgemäß wird nun bei dem erfindungsgemäßen Verfahren mindestens ein Parameter bestimmt, der die Haltbarkeit (MHD/Verbrauchsdatum/Einfrierdatum) des Lebensmittels/Verpackungsgut beeinflusst und abhängig davon ein verpackungsindividuelles MHD/Verbrauchsdatum/Einfrierdatum errechnet. Das erfindungsgemäße Verfahren trägt dem Umstand Rechnung, dass das MHD/Verbrauchsdatum/Einfrierdatum von Produktions- und/oder Verpackungsparametern abhängt. Diese können sich von Verpackung zu Verpackung unterscheiden, was bei dem verpackungsindividuellen oder formatindividuellen MHD/Verbrauchsdatum/Einfrierdatum berücksichtigt wird.

Ein Produktionsparameter ist beispielsweise die Temperatur im Kern des Lebensmittels, die beispielsweise bei einer Wärmebehandlung erzielt worden ist. Andere Parameter sind beispielsweise die Dauer der Wärmebehandlung und/oder die Strahlungsbehandlung, z. B. mit UV-Licht. Typische Parameter stammen beispielsweise aus Gefahrenanalysen kritischer Kontroll- und/oder Lenkungspunkte, dem sogenannten HACCP-Konzept (Hazard Analysis Critical Control Points concept), beispielsweise eine Keimbelastung und/oder Keimanzahl. Bei der Verpackung ist ein relevanter Parameter beispielsweise die Konzentration einer Komponente, beispielsweise die Sauerstoffkonzentration in der Atmosphäre der Verpackung. Vorzugsweise wird diese verpackungsindividuell vorzugsweise zerstörungsfrei bestimmt. Mindestens einer dieser Parameter wird bei der Ermittlung des MHD/Verbrauchsdatum/ Einfrierdatum verpackungsindividuell berücksichtigt.

Die Zusammensetzung des Schutzgases kann anhand der gemessenen Keimanzahl variiert werden, um eine daraus resultierende verbesserte Keimtötung durch Schutzgaskomponenten zu erreichen.

Das Wählen eines Parameters und dessen Anpassung könnte auch per Computer geschehen bzw. einen Regelkreis geschehen

Das ermittelte MHD/Verbrauchsdatum/Einfrierdatum wird auf jeder Verpackung festgehalten, z. B. aufgedruckt und/oder in einem Code, beispielsweise einem QR-Code verschlüsselt aufgedruckt und/oder abgespeichert, beispielsweise in einem RFID-Chip, und/oder vorzugsweise in einem Computersystem verpackungsindividuell oder formatindividuell abgespeichert. Formatindividuell im Sinne der Erfindung bedeutet, dass die MHDs/Verbrauchsdaten/Einfrierdaten eines Formates, d.h. der Verpackungen, die gleichzeitig hergestellt werden, identisch sind oder in dem Fall, dass unterschiedliche Produkte in einem Format enthalten sind, dass die MHDs/Verbrauchsdaten/Einfrierdaten von jeweils gleichen Produkten identisch sind.

Vorzugsweise wird bei der Bestimmung des MHDs/Verbrauchsdatums/Einfrierdatums ein Parameter des Lebensmittels oder des sensiblen Verpackungsguts berücksichtigt, beispielsweise das Ausgasverhalten. Befindet sich beispielsweise Luft in dem Produkt, die langsam entweicht, kann dies eine beim Verpacken erzielte im Vergleich zu Luft reduzierte Sauerstoffkonzentration wieder anheben. Dies wird vorzugsweise bei der Bestimmung des MHDs/Verbrauchsdatums/Einfrierdatums berücksichtigt. Gegebenenfalls muss der MHD/Verbrauchsdatum/Einfrierdatum bei einer weiteren Messung noch einmal korrigiert werden.

Der Gegenstand der vorliegenden Erfindung ist ein Verfahren zur Überprüfung des MHD/Verbrauchsdatum/Einfrierdatum eines verpackten Lebensmittels oder sensiblen Gutes, bei dem die Konzentration mindestens einer Komponente der Gasphase der Verpackung vorzugsweise zerstörungsfrei gemessen und daraus ein MHD/Verbrauchsdatum errechnet wird.

Bei diesem Gegenstand der vorliegenden Erfindung ist ein MHD/Verbrauchsdatum, insbesondere nach der Fertigstellung der Verpackung und/oder vor dem Versand ermittelt worden. Diese Verpackung wird dann zu einem Händler transportiert, gegebenenfalls gelagert und dann in einem Ladenlokal zum Verkauf angeboten. Der Käufer lagert die Verpackung in der Regel noch für eine gewisse Zeit bei sich, bevor der Inhalt der Verpackung verbraucht wird. Erfindungsgemäß wird nun während dieser Versorgungskette die Konzentration mindestens einer Komponente der Gasphase der Verpackung vorzugsweise zerstörungsfrei gemessen und daraus ein neues MHD/Verbrauchsdatum berechnet oder das alte bestätigt.

Vorzugsweise erfolgt diese Messung in der Verpackung beim Verlassen der Herstellerfirma. Alternativ oder zusätzlich erfolgt die Messung:
- beim Händler vor dem Versand,
- beim Einlegen der Verpackung in ein Verkaufsregal,
- nach einer bestimmten Verweilzeit der Verpackung im Regal und/oder
- beim Kunden.

Vorzugsweise erfolgt die Messung der Konzentration der Komponente in der Gasphase mit einem mobilen Gerät und vorzugsweise zerstörungsfrei. Basierend auf diesen Daten wird dann das MHD/Verbrauchsdatum bestätigt oder korrigiert und das korrigierte MHD/Verbrauchsdatum verpackungsindividuell notiert, beispielsweise auf einem Tag, beispielsweise einem RFID, der an der Verpackung angeordnet ist. Vorzugsweise wird jede Überprüfung beispielsweise elektronisch registriert und das neue oder alte MHD/Verbrauchsdatum gemeinsam mit dem Überprüfungsdatum festgehalten. Vorzugsweise ist das mobile Gerät drahtlos mit einem Smartphone verbunden, das beispielsweise das MHD/Verbrauchsdatum/Einfrierdatum anzeigen kann und/oder das mobile Gerät steuern kann und/oder Daten zwischen dem mobilen Gerät und einem zentralen Speicher austauschen kann.

Vorzugsweise wird die Konzentration mehrfach innerhalb einer Versorgungskette oder Transportkette gemessen.

Erfindungsgemäß werden bei der Berechnung des MHDs/Verbrauchsdatums/Einfrierdatums Transport- und/oder Lagerbedingungen berücksichtigt. Beispielsweise kann die Temperatur, insbesondere die Maximaltemperatur, registriert werden, der die jeweilige Verpackung innerhalb der Transportkette ausgesetzt wurde. Alternativ oder zusätzlich kann gemessen werden welcher Strahlung die Verpackung bis zu deren Verkauf oder auch noch darüber hinaus ausgesetzt wurde.

Im Folgenden werden die Erfindungen anhand der Figuren 1 - 5 erläutert. Diese Erläuterungen sind lediglich beispielhaft und schränken den allgemeinen Erfindungsgedanken nicht ein. Die Erläuterungen gelten für alle Erfindungsgedanken gleichermaßen.
- **Figur 1**: zeigt die Verpackungsmaschine mit einem Sensor
- **Figuren 2**: zeigt eine Verpackung mit einem Gaskonzentrationsindikatorsubstanzpunkt
- **Figur 3**: zeigt ein Format von Verpackungen
- **Figur 4**: zeigt eine Herstellungs- und Verpackungslinie
- **Figur 5**: zeigt ein Diagramm zur Ermittlung des MHDs

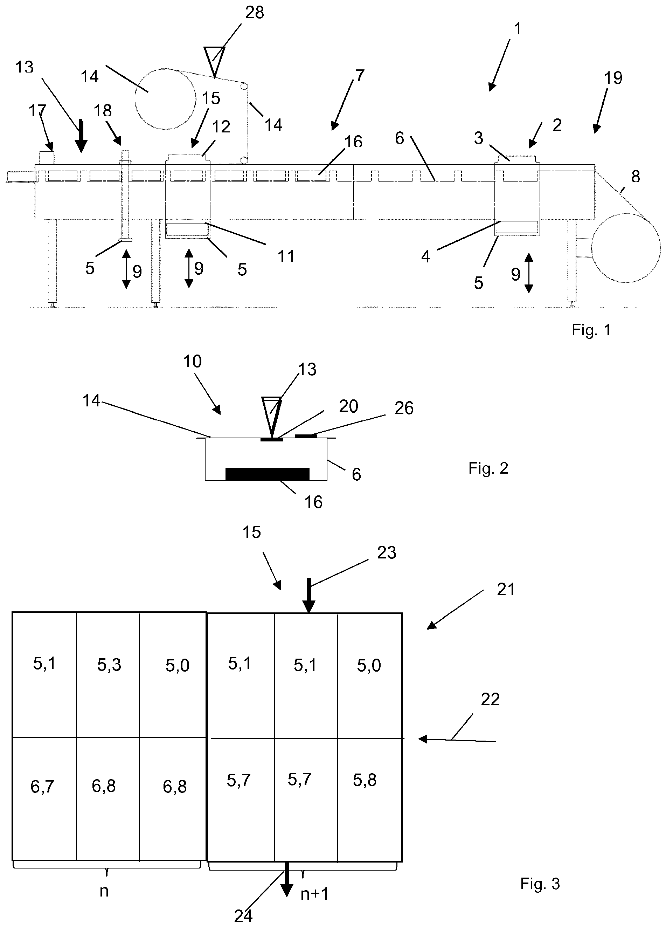

**Figur 1** zeigt eine Verpackungsmaschine 1, die eine Tiefziehstation 2, eine Füllstation 7 sowie eine Siegelstation 15 aufweist. Eine Kunststofffolienbahn 8, die sogenannte Unterfolienbahn, wird von einer Vorratsrolle abgezogen und, vorzugsweise taktweise, entlang der erfindungsgemäßen Verpackungsmaschine hier von rechts nach links transportiert. Bei einem Takt wird die Folienbahn um eine Formatlänge weitertransportiert. Dafür weist die Verpackungsmaschine zwei Transportmittel (nicht dargestellt), in dem vorliegenden Fall jeweils zwei Endlosketten auf, die rechts und links von der Folienbahn angeordnet sind. Jede Endloskette weist Haltemittel auf, die jeweils mit einer Kante der Folienbahn zusammenwirken. Sowohl am Anfang als auch am Ende der Verpackungsmaschine ist für jede Kette jeweils mindestens ein Zahnrad vorgesehen, um das die jeweilige Kette umgelenkt wird. Mindestens eines dieser Zahnräder ist angetrieben. Die Zahnräder im Einlaufbereich 19 und/oder im Auslaufbereich können miteinander, vorzugsweise durch eine starre Welle, verbunden sein. Jedes Transportmittel weist eine Vielzahl von Klemmmitteln auf, die die Unterfolienbahn 8 im Einlaufbereich klemmend ergreifen und die Bewegung des Transportmittels auf die Unterfolienbahn 8 übertragen. Im Auslaufbereich der Verpackungsmaschine wird die klemmende Verbindung zwischen dem Transportmittel und der Unterfolienbahn wieder gelöst. In der Tiefziehstation 2, die über ein Oberwerkzeug 3 und ein Unterwerkzeug 4 verfügt, das die Form der herzustellenden Verpackungsmulde aufweist, werden die Verpackungsmulden 6 in die Unterfolienbahn 8 eingeformt. Das Unterwerkzeug 4 ist auf einem Hubtisch 5 angeordnet, der, wie durch den Doppelpfeil symbolisiert wird, vertikal verstellbar ist. Vor jedem Folienvorschub wird das Unterwerkzeug 4 abgesenkt und danach wieder angehoben. Im weiteren Verlauf der Verpackungsmaschine werden die Verpackungsmulden dann in der Füllstation 7 mit dem Verpackungsgut 16 gefüllt. In der sich daran anschließenden Siegelstation 19, die ebenfalls aus einem Oberwerkzeug 12 und einem vertikal verstellbaren Unterwerkzeug 11 besteht, wird eine Oberfolienbahn auf die Verpackungsmulde gesiegelt. Auch in der Siegelstation werden das Oberwerkzeug und/oder das Unterwerkzeug vor und nach jedem Folientransport abgesenkt bzw. angehoben. Auch die Oberfolienbahn 14 kann tiefgezogen und/oder in Transportmitteln geführt sein bzw. von Transportketten transportiert werden, wobei sich diese Transportmittel dann nur von der Siegelstation und ggf. stromabwärts erstrecken. Ansonsten gelten die Ausführungen, die zu den Transportmitteln der Unterfolienbahn gemacht wurden. In der Siegelstation findet vorzugsweise ein Gasaustausch statt, um beispielsweise den Sauerstoffgehalt der Atmosphäre in der Verpackung zu reduzieren. Im weiteren Verlauf der Verpackungsmaschine werden auch die fertiggestellten Verpackungen vereinzelt, was mit dem Schneidwerkzeugen 17, 18 erfolgt. Das Schneidwerkzeug 18 ist in dem vorliegenden Fall ebenfalls mit einer Hubeinrichtung 9 anhebbar bzw. absenkbar. Der Fachmann erkennt, dass bei einem Takt vorzugsweise mehrere Verpackungsmulden tiefgezogen, befüllt und verschlossen werden.

Die Verpackungsmaschine weist zumindest ein Messgerät, beispielsweise einen Sensor 13 auf, mit dem ein Parameter der Verpackung ermittelt werden kann. Beispielsweise liest der Sensor 13 einen Gaskonzentrationsindikatorsubstanzpunkt 20 und/oder einen Druckindikator innerhalb der Verpackung vorzugsweise zerstörungsfrei aus und kann dadurch die Konzentration, beispielsweise die Sauerstoffkonzentration in der Verpackung feststellen oder das Vorhandensein eines bestimmten Drucks feststellt. Vorzugsweise ist ein Sensor pro Packung oder ein Sensor für mehrere Packungen, beispielsweise für eine Reihe oder eine Spalte eines Formates oder für ein gesamtes Format vorhanden.

Weiterhin bevorzugt kann die Verpackungsmaschine 1 ein Bedruckungsmittel 28 aufweisen, das einen oder mehrere Gaskonzentrationspunkte auf die Folienbahn 14 druckt. Vorzugsweise erfolgt der Druck während sich die Folienbahn 14 bewegt.

Figur 2 zeigt beispielhaft eine fertiggestellte Verpackung 10 mit einem Gaskonzentrationsindikatorpunkt 20, der in dem vorliegenden Fall an der dem Verpackungsinneren zugewandten Seite der Deckelfolie 14 angeordnet ist. Ein Sensor 13 kann beispielsweise von dem Gaskonzentrationsindikatorpunkt abgestrahlte elektromagnetische Strahlung analysieren und dadurch Informationen über die Konzentration einer Komponente der Atmosphäre in der Verpackung, beispielsweise die Sauerstoffkonzentration, erhalten. Der Fachmann versteht, dass eine derartige zerstörungsfreie Messung einer Konzentration der Atmosphäre in der Verpackung auch mit anderen Messverfahren erfolgen kann. Der Sensor 13 kann entlang der gesamten Versorgungskette vorhanden sein und die Konzentration einer Komponente in der Gasphase der Verpackung bestimmen. Beispielsweise kann es sich bei dem Sensor auch um ein mobiles Gerät handeln, mit dem ein Spediteur oder ein Händler Messungen machen kann. Vorzugsweise weist die Verpackung ein Etikett oder ein Tag, beispielsweise ein RFID auf, auf dem das MHD und vorzugsweise andere Herstellungs- und/oder Verpackungsrelevante Daten gespeichert sind. Dieses Tag wird vorzugsweise von dem Sensor ausgelesen und nachdem die Messung des Sensors erfolgt ist, wird ein neues MHD auf dem Tag abgespeichert. Der Sensor 13 kann auch mit einem Smartphone oder einem PC, insbesondere einem Tablett-PC verbunden werden, der den Sensor gegebenenfalls steuert und/oder um die Verbindung zu einem zentralen Datenspeicher herzustellen, der Daten zur Ermittlung des neuen MHD enthalten kann und/oder auf dem das neue MHD abgespeichert wird. Diese bevorzugte Sudführungsform ist insbesondere für mobile Sensoren 13 interessant.

Figur 3 zeigt einen Teil einer Verpackungsmaschine und insbesondere zwei produzierte bzw. in Produktion befindliche Formate an Verpackungen. In dem vorliegenden Fall wird bei jedem Takt ein Format von 3 x 2 Verpackungen erzeugt; D.h. das Format weißt in dem vorliegenden Fall 3 Spalten und 2 Reihen auf. Der Fachmann versteht, dass auch ein anderes Format mit einer anderen Anzahl an Reihen und/oder Spalten produziert werden kann. Bei jeder Verpackung des Formats wird eine Konzentration derselben Substanz in der Atmosphäre der Verpackung gemessen, in dem vorliegenden Fall die Sauerstoffkonzentration. Beispielhafte Messwerte sind im Bereich der Verpackung angegeben. Vorzugsweise weißt die nicht erfindungsgemäße Vorrichtung ein Display auf, das die Konzentration der interessierenden Komponente in jeder Verpackung für den Betreiber anzeigt.

Diese Einzelmessungen jeder Verpackung können nun zur Berechnung eines verpackungsindividuellen MHDs herangezogen werden. Je niedriger der gemessene Wert, desto länger das MHD. Des Weiteren können die gemessen Werte herangezogen werden, um die Verpackungsmaschine, beispielsweise deren End- und Begasung, so zu steuern, dass ein bestimmter MHD erzielt wird. Bei der Darstellung gemäß Figur 3 sind 2 Formate, nämlich n und n+1 dargestellt. Das Format n+1 befindet sich noch in der Siegelstation 15, während das Format n bereits stromabwärts von der Siegelstation vorgesehen ist. Die Bewegungsrichtung der Verpackungen ist durch den Fall 22 dargestellt. Die Siegelstation 15 weist in dem vorliegenden Fall eine Absaugung 24 auf, die Gas, insbesondere Luft, aus den jeweiligen Verpackungsmulden absaugt und dadurch einen Unterdruck zwischen der Verpackungsmulde und der Oberfolie, die während des Absaugens zumindest noch nicht vollständig an die Verpackungsmulde gesiegelt ist, erzeugt. Des Weiteren weißt die Vorrichtung in dem vorliegenden Fall eine Begasung 23, insbesondere mit einem von Luft unterschiedlichen Gas, auf. Dabei kann es sich beispielsweise um eine mit N2 und / oder CO2 angereichertes Gasgemisch handeln. In dem vorliegenden Fall soll eine Sauerstoffkonzentration ≤ 6,0 % erzielt werden, damit das gewünschte MHD erreicht wird. Wie man den Messwerten beim Format n entnehmen kann, ist dies bei den Verpackungen, die vergleichsweise weit von der Begasung 23 entfernt sind, nicht gelungen. Auf Grund des Signals dieser Messung wird dann beispielsweise der Volumenstrom und/oder der Druck der Begasung 23 erhöht, und/oder der durch die Absaugung erzeugte Unterdruck erhöht, um auch in diesen Verpackungen die gewünschte Konzentration der Sauerstoffkomponente zu erzielen, was bei dem Format n+1, wie die Messwerte zeigen, auch gelungen ist.

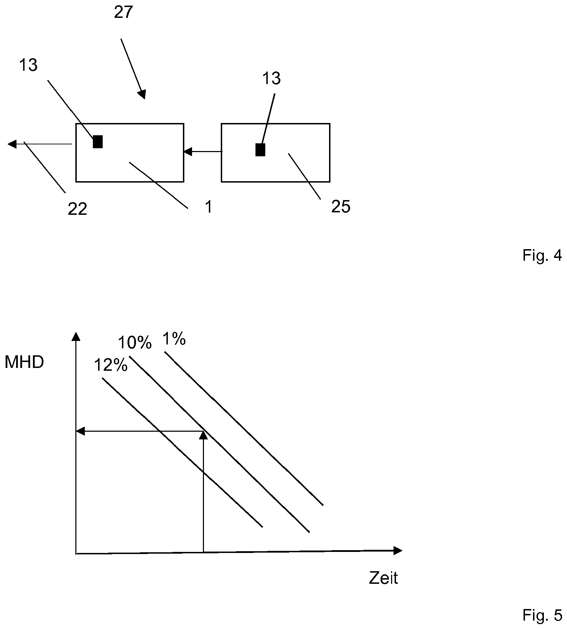

Figur 4 zeigt noch einen erfindungsgemäßen Gegenstand der vorliegenden Erfindung. In dem vorliegenden Fall ist eine Linie 27 dargestellt, die neben einer Verpackungsmaschine 21 noch eine weitere stromaufwärts befindliche Komponente aufweist. In dem vorliegenden Fall weist die Verpackungsmaschine 1 einen Sensor 13 auf, der beispielsweise mindesten die Konzentration einer Komponente in der Verpackung misst. Des Weiteren weist die Linie 27 eine weitere Komponente 25 auf, die ebenfalls mit einem Sensor 13 versehen ist, der einen Herstellungsparameter ermittelt. Die Messung zumindest eines dieser Sensoren 13 wird bei der Ermittlung des MHDs ermittelt. Der Sensor der Verpackungsmaschine kann beispielsweise die Sauerstoffkonzentration in der Atmosphäre der Verpackung messen. Bei der Komponente 25 kann es sich einen Kutter, insbesondere einen Vakuumkutter handeln. Der Sensor 13 misst in diesem Fall vorzugsweise den Unterdruck, der beim Kuttern anliegt. Durch den Unterdruck beim Kuttern wird das Ausgasverhalten des resultierenden Produkts beeinflusst. Basierend auf den Messdaten von einem oder vorzugsweise beiden Sensoren wird dann das MHD berechnet.

Figur 5 zeigt ein Diagramm anhand dessen das MHD ermittelt werden kann, das auf der Y-Achse aufgetragen ist. Auf der X-Achse ist die Zeit aufgetragen, die seit der Herstellung der Verpackung vergangen ist. Des Weiteren weist das Diagramm eine Kurvenschar auf, wobei jede Kurve hier einer Sauerstoffkonzentration in der Atmosphäre der Verpackung entspricht. In dem vorliegenden Fall hat der Sensor 13 eine Konzentration von 10 % Sauerstoff nach einer gewissen Anzahl von Tagen ermittelt daraus kann ein MHD ermittelt werden.

### Bezugszeichenliste:

- 1: Verpackungsmaschine
- 2: Tiefziehstation
- 3: Oberwerkzeug der Tiefziehstation
- 4: Unterwerkzeug der Tiefziehstation
- 5: Hubtisch, Träger eines Werkzeugs der Siegel- , Tiefziehstation und/oder der Schneideinrichtung
- 6: Verpackungsmulde
- 7: Füllstation
- 8: Unterfolienbahn
- 9: Hubeinrichtung
- 10: fertiggestellte Verpackung
- 11: Unterwerkzeug der Siegelstation
- 12: Oberwerkzeug der Siegelstation
- 13: Sensor, Sauerstoffsensor
- 14: Oberfolie
- 15: Siegelstation
- 16: Verpackungsgut
- 17: Längsschneider
- 18: Querschneider
- 19: Einlaufbereich
- 20: Gaskonzentrationsindikatorsubstanzpunkt, Sauerstoffkonzentrationsindikatorsubstanzpunkt
- 21: Format von Verpackungen, hier 3 x 2
- 22: Transportrichtung der Verpackungsfolie und/oder des Verpackungsguts
- 23: Begasung
- 24: Absaugung
- 25: stromaufwärtige Komponente
- 26: Etikett, Tag, RFID
- 27: Linie
- 28: Bedruckungsmittel

## Patentansprüche

1. Verfahren zur Überprüfung des Mindesthaltbarkeitsdatums eines verpackten Lebensmittels oder sensiblen Gutes, wobei die Konzentration mindestens einer Komponente der Gasphase der Verpackung, vorzugsweise zerstörungsfrei, gemessen und daraus ein verpackungsindividuelles Mindesthaltbarkeitsdatum errechnet wird, und wobei bei der Berechnung des Mindesthaltbarkeitsdatums Transport- und/oder Lagerbedingungen berücksichtigt werden, **dadurch gekennzeichnet, dass** bei der Berechnung außerdem Produktions- und Verpackungsparameter berücksichtigt werden.

2. Verfahren nach Anspruch 1, **dadurch gekennzeichnet, dass** die Konzentration mit einem mobilen Gerät ermittelt wird.

3. Verfahren nach einem der Ansprüche 1 oder 2, **dadurch gekennzeichnet, dass** das Mindesthaltbarkeitsdatum mindestens einmal, vorzugsweise bei jeder Bestimmung, insbesondere auf der Verpackung, aufgedruckt und/oder gespeichert wird.

4. Verfahren nach einem der Ansprüche 1 - 3, **dadurch gekennzeichnet, dass** die Konzentration mehrfach innerhalb einer Versorgungskette gemessen wird.

5. Verfahren nach einem der voranstehenden Ansprüche, **dadurch gekennzeichnet, dass** die Zusammensetzung der Gasphase in Abhängigkeit des Messwerts, vorzugsweise automatisch, verändert wird.

## Claims

1. Method for checking the best before date of a packaged food or sensitive product, wherein the concentration of at least one component of the gas phase of the package is measured, preferably non-destructively, and a best before date specific to the individual package is calculated therefrom, and wherein transport and/or storage conditions are taken into account in the calculation of the best before date, **characterized in that** production and packaging parameters are also taken into account in the calculation.

2. Method according to Claim 1, **characterized in that** the concentration is determined with a mobile device.

3. Method according to either of Claims 1 and 2, **characterized in that** the best before date is printed, at least once, preferably for each determination, in particular on the package, and/or is stored.

4. Method according to one of Claims 1-3, **characterized in that** the concentration is measured a number of times within a supply chain.

5. Method according to one of the preceding claims, **characterized in that** the composition of the gas phase is changed in dependence on the measured value, preferably automatically.

## Revendications

1. Procédure de vérification de la date de conservation minimale d'un aliment emballé ou d'un produit sensible, la concentration d'au moins un composant de la phase gazeuse de l'emballage étant mesurée, de préférence de manière non destructive, et une date de conservation minimale spécifique à l'emballage étant calculée à partir de ladite concentration, et les conditions de transport et/ou de stockage étant prises en compte lors du calcul de la date de conservation minimale, **caractérisé en ce que** les paramètres de production et d'emballage sont également pris en compte dans le calcul.

2. Procédé selon la revendication 1, **caractérisé en ce que** la concentration est déterminée à l'aide d'un appareil mobile.

3. Procédé selon l'une des revendications 1 ou 2, **caractérisé en ce que** la date de conservation minimale est imprimée au moins une fois, de préférence à chaque détermination, notamment sur l'emballage, et/ou mémorisée.

4. Procédé selon l'une des revendications 1 à 3, **caractérisé en ce que** la concentration est mesurée plusieurs fois à l'intérieur d'une chaîne d'alimentation.

5. Procédé selon l'une des revendications précédentes, **caractérisé en ce que** la composition de la phase gazeuse est modifiée en fonction de la valeur de mesure, de préférence automatiquement.
